(19) Europäisches Patentamt
European Patent Office
Office européen des brevets

(11) EP 1 397 376 B1

(12) EUROPÄISCHE PATENTSCHRIFT

(45) Veröffentlichungstag und Bekanntmachung des Hinweises auf die Patenterteilung:
**04.05.2005 Patentblatt 2005/18**

(21) Anmeldenummer: **02727608.8**

(22) Anmeldetag: **03.06.2002**

(51) Int Cl.[7]: **C07H 15/203**

(86) Internationale Anmeldenummer:
**PCT/EP2002/006070**

(87) Internationale Veröffentlichungsnummer:
**WO 2002/102816 (27.12.2002 Gazette 2002/52)**

(54) **VERFAHREN ZUR HERSTELLUNG VON PERBENZYLIERTEN 1-O-GLYCOSIDEN**
METHOD FOR PRODUCING PERBENZYLATED 1-O-GLYCOSIDES
PROCEDE POUR LA PRODUCTION DE 1-O-GLUCOSIDES PERBENZYLES

(84) Benannte Vertragsstaaten:
**AT BE CH CY DE DK ES FI FR GB GR IE IT LI LU MC NL PT SE TR**

(30) Priorität: **19.06.2001 DE 10129888**

(43) Veröffentlichungstag der Anmeldung:
**17.03.2004 Patentblatt 2004/12**

(73) Patentinhaber: **SCHERING AKTIENGESELLSCHAFT**
**13342 Berlin (DE)**

(72) Erfinder:
- **PLATZEK, Johannes**
  **12621 Berlin (DE)**
- **NIEDBALLA, Ulrich**
  **14195 Berlin (DE)**
- **GRASKE, Klaus-Dieter**
  **12169 Berlin (DE)**

(56) Entgegenhaltungen:
**EP-A- 0 882 733** **WO-A-01/68659**
**US-A- 5 874 411**

- **SCHMIDT R R: "NEUE METHODEN ZUR GLYCOSID- UND OLIGOSACCHARIDSYNTHESE - GIBT ES ALTERNATIVEN ZUR KOENIGS-KNORR-METHODE?" ANGEWANDTE CHEMIE, VCH VERLAGSGESELLSCHAFT, WEINHEIM, DE, Bd. 98, 1986, Seiten 213-236, XP001018622 ISSN: 0044-8249**
- **SUGAWARA T ET AL: "SYNTHESIS OF OMEGA-(METHOXYCARBONYL)ALKYL AND 9-(METHOXYCARBONYL)-3,6-DIOXANONYL GLYCOPYRANOSIDES FOR THE PREPARATION OF CARBOHYDRATE-PROTEIN CONJUGATES" CARBOHYDRATE RESEARCH, ELSEVIER SCIENTIFIC PUBLISHING COMPANY. AMSTERDAM, NL, Bd. 230, Nr. 1, 1992, Seiten 117-150, XP001018683 ISSN: 0008-6215**
- **SCHMIDT R R ET AL: "1-O-ALKYLATION OF D-GLUCOPYRANOSE" JOURNAL OF CARBOHYDRATE CHEMISTRY, NEW YORK, NY, US, Bd. 3, Nr. 1, 1984, Seiten 67-84, XP001021740 ISSN: 0732-8303**
- **LOCKHOFF OSWALD: "An access to glycoconjugate libraries through multicomponent reactions" ANGEWANDTE CHEMIE. INTERNATIONAL EDITION, VERLAG CHEMIE. WEINHEIM, DE, Bd. 37, Nr. 24, 1998, Seiten 3436-3439, XP002171115 ISSN: 0570-0833**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 397 376 B1

Printed by Jouve, 75001 PARIS (FR)

## Beschreibung

Die Erfindung betrifft ein neues Verfahren zur Herstellung von perbenzylierten 1-O-Glycosiden der allgemeinen Formel I, das in den Patentansprüchen näher gekennzeichnet ist. Das erfindungsgemäße Verfahren geht von kostengünstigen Ausgangsmaterialien aus, liefert gute Ausbeuten und erlaubt die Herstellung von perbenzylierten Sacchariden mit 1-O-funktionalisierten Seitenketten im großen Maßstab.

Perbenzylierte Saccharid-Derivate sind wertvolle Zwischenprodukte in der Synthesechemie. Vor allem die pharmazeutische Chemie verwendet derartige Bausteine sehr häufig, da viele hochpotente und selektive Pharmaka Zuckerreste tragen. So sind beispielsweise im Journal of Drug Targeting 1995, Vol. 3, pp. 111-127 Anwendungen des sogenannten "Glycotargetings" beschrieben. Sogenannte "multi-antennary sugar chains" sind in Chemistry Letters 1998, S. 823 beschrieben. Durch Clustering von Zuckereinheiten wird die Carbohydrat-Rezeptor-Wechselwirkung bei der Zell-Zell-Interaktion wesentlich verbessert. Die Synthese von Galaktosiden mit hoher Affinität zum Asialoglycoprotein Receptor ist im J. Med. Chem. 1995, 38, p. 1538 publiziert worden (siehe auch Int. J. Peptide Protein Res. 43, 1994, p. 477). Hier werden derivatisierte Galactosen mit funktionalisierten Seitenketten hergestellt, die anschließend an verschiedene andere Moleküle gehängt werden können. Eine gute Übersicht über die Verwendung von Sacchariden als Basis der Glycobiologie ist in Acc. Chem. Res. 1995, 321 gegeben worden . Auch für die Synthese von LewisX Mimetika (Tet. Lett. Vol. 31, 5503) dienen funktionalisierte Monosaccharide als Vorstufen (siehe auch JACS 1996, 118, 6826).

Die Verwendung von derivatisierten Monosacchariden als Zwischenstufen für potentielle Pharmazeutika ist in Current Medicinal Chemistry, 1995, 1, 392 gut dargestellt worden . Perbenzylierte-1-OH-Zucker-Derivate (Galactose, Glucose) werden auch in der Synthese von herzaktiven Glycosiden (Digitoxin-Konjugate) eingesetzt. Die 1-O-Glycosidierung erfolgt hier via Trichloracetimidat und $BF_3$-Katalyse (J. Med. Chem. 1986, 29, p. 1945). Zur Herstellung immobilisierter Zucker-Liganden (z. B. Verknüpfung an HSA) werden funktionalisierte, geschützte Monosaccharide eingesetzt (Chemical Society Reviews 1995, p. 413).

Ziel einer Gruppe von Synthesen ist es, über eine 1-O-Glycosidierungsreaktion zusätzlich Funktionalität ins Zuckermolekül einzuführen. Hier sind vor allem endständige COOH-, Amino- oder OH-Gruppen von Interesse, da diese in Folgeschritten weiter umgesetzt werden können.

Die Herstellung von 1-O-Glycosiden erfolgt in den meisten Fällen nach klassischen Methoden, wie z.B. nach der von Koenigs-Knorr, Helferich oder der von R.R. Schmidt beschriebenen Trichloracetimidat-Methode [W. Koenigs und E. Knorr, Ber. dtsch. chem. Ges. 34 (1901) 957; B. Helferich u. J. Goendeler, Ber. dtsch. Chem. Ges. 73, (1940) 532; B. Helferich, W. Piel und F. Eckstein, Chem. Ber. 94 (1961), 491; B. Helferich u. W.M. Müller, Chem. Ber. 1970, 103, 3350; G. Wulff, G. Röhle und W. Krüger, Ang. Chem. Internat. Edn., 1970, 9, 455; J. M. Berry u. G.G.S. Duthon, Canad. J. Chem. 1972, 50, 1424; R. R. Schmidt, Angew. Chem. 1986, 98, 213.] .

Allen diesen Methoden ist gemeinsam, daß die 1-Hydroxylgruppe in eine reaktive Form überführt wird, die letztlich als Abgangsgruppe dient. Unter Lewissäure-Katalyse (teilweise in stöchiometrischer Menge), erfolgt die eigentliche Umsetzung mit einem Alkohol zum 1-O-Glycosid. Für derartige Umsetzungen gibt es zahlreiche Beispiele in der Literatur.

So ist bei der Herstellung des Immunostimulans KRN-7000 (Kirin Brewery) die Kondensation von Tetra-O-benzyl-β-D-galactopyranosyl-bromid mit einem primären Alkohol, dessen Hydroxylgruppe am Ende einer di-Hydroxy-Amido-C-Kette sitzt (in DMF/Toluol unter Lewissäure Katalyse) ein zentraler Schritt (Drug of the Future 1997, 22(2), p. 185). In dem japanischen Patent JP 95-51764 ist die Umsetzung von 1-O-Acetyl-2,3,4-tri-0-benzyl-L-fucopyranose mit Polyoxyethylen-30-Phytosterol (BPS-30, NIKKO Chem., Japan) unter Trimethylsilylbromid/Zinktriflat-Katalyse beschrieben worden. In Bull. Chem. Soc. 1982, 55(4), p. 1092-6 sind 1-O-Glycosidierungen von Perbenzyl-Zuckern unter Titantetrachlorid-Katalyse in Dichlormethan beschrieben.

In Liebigs Ann. Org. Bioorg. Chem.; EN; 9; 1995; 1673-1680 ist die Herstellung von 3,4,5-Trisbenzyloxy-2-benzyloxymethyl-6-(2-hexadecyloxyethoxy)-tetrahydropyran beschrieben. Ausgehend von 2,3,4,6-Tetra-O-benzyl-D-glucopyranose wird die 1-O-Glycosidierung unter Verwendung von $Bu_4NBr$, $CoBr_2$, $Me_3SiBr$ und Molekularsieb in Methylenchlorid innerhalb von 60 Stunden durchgeführt .

Ein Tetrabenzylderivat, das eine endständige, als Methylester geschützte Carboxylgruppe enthält, ist in Carbohydr. Res.; EN; 230; 1; 1992; 117 beschrieben. Die Carboxylgruppe kann danach freigesetzt und weiter umgesetzt werden. Zur Glycosidierung wird Silbercarbonat in Dichlormethan verwendet. Die Verwendung des teuren Silbercarbonats limitiert die Ansatzgröße und macht ein wirtschaftliches up-scaling fast unmöglich. Das gleiche Problem gilt für die nachfolgende Verbindung, die in Tetrahedron Lett. 30, 44, 1989, p. 6019 beschrieben worden ist. Hier wird 2,3,4,6-Tetra-O-benzyl-D-mannosyl-bromid in Nitromethan mit 2-Benzyloxyethanol unter Zurhilfenahme von Quecksilbercyanid zum 1-O-Glycosid umgesetzt. Die Verwendung von Quecksilbercyanid in pilot-plant-Anlagen ist problematisch und aus umweltpolitischer Sicht abzulehnen.

Die in neuester Zeit beschriebenen Substanzbibliotheken für das Hochdurchsatz-Screening verwenden sehr häufig Saccharide (Angew. Chemie 1995, 107, 2912). Hier ist es das Ziel, Zuckerbausteine in geschützter Form vorliegen zu haben, die eine funktionelle Gruppe, wie z.B. -COOH, oder $-NH_2$ tragen, welche z.B. in einer automatisierten Synthese umgesetzt werden können. Die Bausteine , die dafür Verwendung finden, sind von Lockhoff, Angew. Chem. 1998, 110(24), S. 3634 beschrieben worden. Vor allem die 1-O-Essigsäure von Perbenzyl-Glucose ist hier von Bedeutung. Die Herstellung erfolgt über 2 Stufen, via Trichloracetimidat und Umsetzung mit Hydroxyessigsäureethylester, $BF_3$-Katalyse in THF und anschließender Verseifung mit NaOH in MeOH/THF. Die Gesamtausbeute über 2 Stufen beträgt allerdings nur 59 %.

In der gleichen Publikation wird auch die Herstellung eines 1-O-(Aminoethyl)-Glycosids der perbenzylierten Glucose beschrieben. Die Umsetzung erfolgt, wieder ausgehend vom Trichloracetimidat, durch Umsetzung mit N-Formylaminoethanol unter $BF_3$-Katalyse in THF und anschließender Verseifung in MeOH/THF. Die Gesamtausbeute ist auch hier relativ gering, sie beträgt 45 %.

Ein 1-0-(Aminoethyl)-Derivat der Perbenzylxylose wird in Carbohydrate Research 1997, 298, p. 173 als Zwischenprodukt durchlaufen. Die Synthese ist jedoch sehr langwierig, da sie vom 1-Brom-peracetat der Xylose startet. Die eigentlliche 1-O-Glycosidierung erfolgt über einen 1-Phenylthioether, der mit 2-Azidoethanol unter DMTST-Katalyse (= Dimethyl (methyltio)-sulfonium-triflat) in Dichlormethan umgesetzt wird (Gesamtstufenzahl: 7). Die Gesamtausbeute ist mit unter 40 % für eine industrielle Anwendung nicht geeignet.

Im Übersichtsartikel von R.R. Schmidt in Angew. Chem. 1986, 98, S. 213-236 werden direkte Umsetzungen von 1-OH-Perbenzylglucose und -ribose mit 2-Halogenestern und Triflaten beschrieben. Als Base wird Natriumhydrid in THF oder Benzol verwendet (Chem. Ber. 1982, 115), die Ausbeuten liegen zwischen 40 und 55 %. Auch der Einsatz von Natriumhydrid in Dioxan oder Kalium-tert.-butylat in THF (beides bei Raumtemperatur) ist zur 1-O-Alkylierung mit Triflaten beschrieben (Angew. Chem. 1986, 98, S. 218). Die strengstens einzuhaltenden wasserfreien Reaktionsbedingungen stellen eine große Hürde beim up-scaling derartiger Alkylierungen dar.

Alle bisher bekannten Verfahren haben den großen Nachteil, daß sich ein up-scaling des Prozesses nicht ohne weiteres bewerkstelligen läßt. Die Verwendung von Lewis-Säuren bei der 1-O-Glycosidierung sowie von Natriumhydrid bei der 1-O-Alkylierung erfordert stets wasserfreie Reaktionsbedingungen, was bei großen Ansätzen immer mit Schwierigkeiten verbunden ist. Auch die Aufarbeitung und Entsorgung der Reaktionshilfsstoffe (Hg/Cyanid/etc.) ist in vielen Fällen ein Problem.

Aufgabe der Erfindung war es deshalb, ein Verfahren bereitzustellen, mit dem perbenzylierte Saccharide mit 1-Ofunktionalisierter Seitenkette in größerem Maßstab, preiswert und umweltfreundlich hergestellt werden können.

Die Aufgabe der Erfindung wird gemäß dem in den Ansprüchen angegebenen Verfahren gelöst, mit welchem perbenzylierte 1-O-Glycoside der allgemeinen Formel I

$$\underset{(OR)_n}{\overset{\displaystyle \text{Zucker}^1}{|}} - O - L - X - H \qquad (I)$$

hergestellt werden können. Gemäß Definition der Erfindung bedeutet $Zucker^1$ in der allgemeinen Formel I ein in 1-OH-Position funktionalisiertes Monosaccharid, wobei es sich hierbei auch um Desoxyzucker handeln kann, die anstelle einer oder mehrerer OH-Gruppen ein H-Atom enthalten. In einer bevorzugten Ausführungsform der Erfindung bedeutet der Zucker in der allgemeinen Formel I ein Monosaccharid mit 5 oder 6 C-Atomen, z. B. Glucose, Mannose, Galactose, Ribose, Arabinose oder Xylose oder deren Desoxyzucker wie beispielsweise 6-Desoxygalactose (Fucose) oder 6-Desoxy-Mannose (Rhamnose).

Der Rest R stellt die Benzylgruppe dar, die in Abhängigkeit vom eingesetzten Monosaccharid oder dessen Desoxyform mindestens zweifach vorhanden ist und beim Einsatz von Di-, Tri- oder Polysacchariden entsprechend mehrfach vorhanden ist.

Der Rest X bedeutet -COO- oder -NH-. Im Ergebnis des erfindungsgemäßen Verfahrens werden also Alkohole, Carbonsäuren oder Amine der allgemeinen Formel I erhalten.

Der Rest L kann eine geradkettige, verzweigte, gesättigte oder ungesättigte $C_1$-$C_{30}$-Kohlenstoffkette bedeuten, die gegebenenfalls unterbrochen ist durch 1-10 Sauerstoffatome, 1-3 Schwefelatome, 1-2 Phenylen-, 1-2 Phenylenoxy-, 1-2 Phenylendioxygruppen, einen Thiophen-, Pyrimidin- oder Pyridinrest und/oder gegebenenfalls substituiert ist mit 1-3 Phenyl-, 1-3 Carboxyl-, 1-5 Hydroxy-, 1-5 O-$C_1$-$C_7$-alkyl-, 1-3 Aminogruppen, 1-3 $CF_3$-Gruppen oder 1-10 Fluoratomen. Im Sinne der Erfindung bevorzugte Reste L sind

$$\gamma - (CH_2)_n - \delta \qquad n=1\text{-}15$$

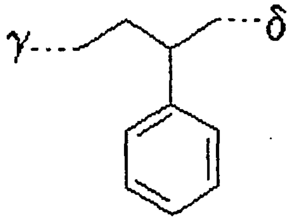

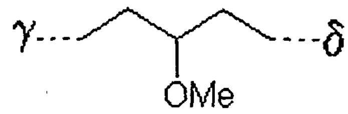

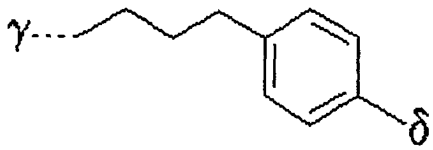

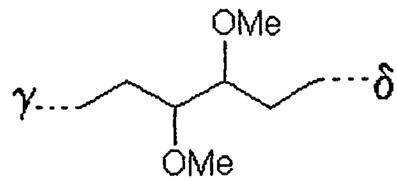

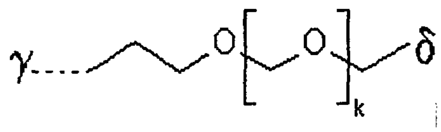

k = 0-9

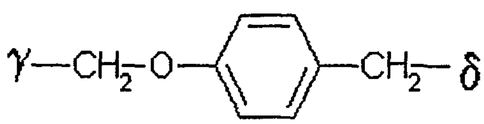

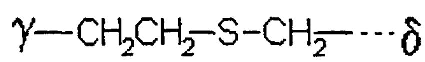

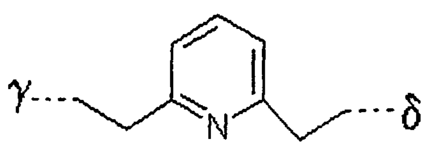

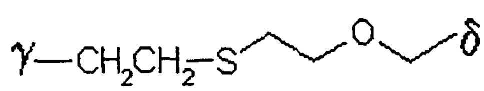

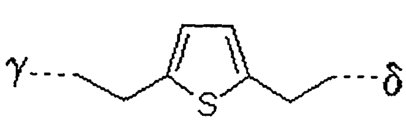

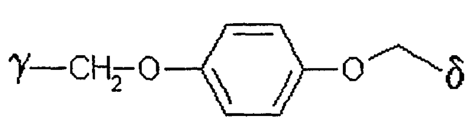

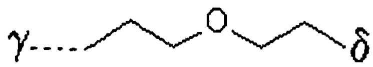

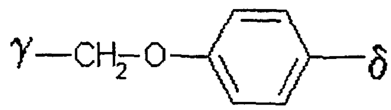

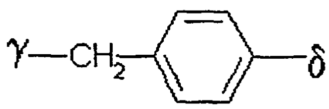

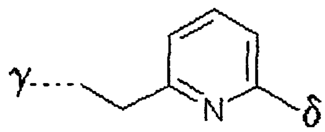

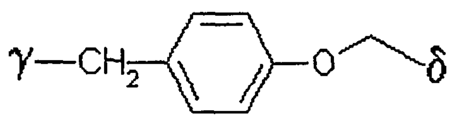

wobei γ die Verknüpfungsstelle am Zucker bedeutet und δ die Verknüpfungsstelle zum Rest X ist. Ein besonders bevorzugter Linker L ist die $-CH_2$-Gruppe.

Zur Herstellung der perbenzylierten 1-O-Glycoside der allgemeinen Formel I wird ein perbenzylierter 1-OH-Zucker der allgemeinen Formel II

Zucker$^1$ - OH
|
$(OR)_n$ (II),

worin Zucker, R und n die oben angegebene Bedeutung haben, in dem organischen Lösungsmittel Diethoxymethan gelöst und mit einem Alkylierungsreagenz der allgemeinen Formel III

Nu - L - X - Sg (III),

worin Nu ein Nucleofug bedeutet, L und X die genannte Bedeutung haben und Sg eine Schutzgruppe ist, in Gegenwart einer Base und gegebenenfalls eines Phasentransfer-Katalysators umgesetzt. Als Nucleofug können im Alkylierungsreagenz der allgemeinen Formel III beispielsweise die Reste - Cl, -Br, -J, -OTs, -OMs, $-OSO_2CF_3$, $-OSO_2C_4F_9$ oder $-OSO_2C_8F_{17}$ enthalten sein.

Bei der Schutzgruppe Sg handelt es sich um eine übliche Säureoder Amin-Schutzgruppe, je nachdem ob X den Rest -COO- oder - NH- bedeutet. Diese Schutzgruppen sind dem Fachmann gut vertraut (Protective Groups in Organic Syntheses, second Edition, T.W.Greene and P.G.M. Wuts, John Wiley & Sons Inc., New York 1991) .

Die erfindungsgemäße Umsetzung kann bei Temperaturen von 0-50°C, vorzugsweise von 0°C bis Raumtemperatur erfolgen. Die Reaktionszeiten betragen von 10 Minuten bis 24 Stunden, vorzugsweise von 20 Minuten bis

12 Stunden.

Die Base wird entweder in fester Form, vorzugsweise fein gepulvert oder flüssig oder als 10-70%ige, vorzugsweise 30-50%ige, wäßrige Lösung zugesetzt. Als bevorzugte Basen dienen NaOH, KOH, Cäsiumcarbonat, Kaliumcarbonat, 1,8-Diazabicyclo-[5.4.0]undec-7-en (DBU), 1,5-Diazabicyclo[2.2.2]octan (DBN), Kalium t butoxid und Natrium t butoxid.

Als erfindungsgemäßes Lösungsmittel wird Diethoxymethan verwendet. Als Phasentransfer-Katalysatoren dienen im erfindungsgemäßen Verfahren die für diesen Zweck bekannten quartären Ammonium- oder Phosphoniumsalze oder auch Kronenether wie z. B. [15]-Krone-5- oder [18]-Krone-6. Vorzugsweise kommen quartäre Ammoniumsalze mit vier gleichen oder verschiedenen Kohlenwasserstoffgruppen am Kation, ausgewählt aus Methyl, Ethyl, Propyl, Isopropyl, Butyl oder Isobutyl in Frage. Die Kohlenwasserstoffgruppen am Kation müssen groß genug sein, um eine gute Löslichkeit des Alkylierungsreagenzes im organischen Lösungsmittel zu gewährleisten. Erfindungsgemäß besonders bevorzugt wird $N(Butyl)_4^+$-$Cl^-$, $N(Butyl)_4^+$-$HSO_4^-$, aber auch $N(Methyl)_4^+$-$Cl^-$ eingesetzt.

Nach erfolgter Umsetzung kann die Aufarbeitung des Reaktionsgemisches durch Isolierung des noch geschützten Endproduktes und nachfolgende übliche Abspaltung der Schutzgruppe zum Endprodukt der allgemeinen Formel I erfolgen. Bevorzugt ist es jedoch, das noch geschützte Endprodukt nicht zu isolieren, sondern das Lösungsmittel zu entfernen, den Rückstand in einem neuen, für die Abspaltung der Schutzgruppe geeigneten Lösungsmittel aufzunehmen und hier die Abspaltung durchzuführen. Die Verfahrensweise zur Abspaltung der Schutzgruppe und zur Regenerierung der Säure-, Amino-, Hydroxy- oder Thiolgruppe ist dem Fachmann gut bekannt.

Handelt es sich z. B. bei der Schutzgruppe Sg um eine Säureschutzgruppe, die das acide Proton der Carboxygruppe blockiert, also z. B. um Methyl, Ethyl, Benzyl oder um tert-Butyl, so wird die Säure üblicherweise durch alkalische Hydrolyse regeneriert. In dem Verfahren der Erfindung wird für diesen Fall nach Entfernung des Lösungsmittels aus der Alkylierungsreaktion nun der Rückstand in einem neuen Lösungsmittel, z. B. Methanol, Ethanol, Tetrahydrofuran, Isopropanol, Butanol oder Dioxan aufgenommen. Es wird dann eine wäßrige Lösung einer Base zugesetzt und bei Temperaturen von 0-100°C die alkalische Hydrolyse durchgeführt.

Als Hydroxyschutzgruppen (in L) kommen z.B. Benzyl-, 4-Methoxybenzyl-, 4-Nitrobenzyl-, Trityl-, Diphenylmethyl-, Trimethylsilyl-, Dimethyl-tert-butylsilyl oder Diphenyl-tertbutylsilylgruppen in Frage.
Die Hydroxygruppen können auch z.B. als THP-Ether, α-Alkoxyethylether, MEM-Ether oder als Ester mit aromatischen oder aliphatischen Carbonsäuren, wie z.B. Essigsäure oder Benzoesäure, vorliegen. Im Fall von Polyolen können die Hydroxygruppen auch in Form von Ketalen mit z. B. Aceton, Acetaldehyd, Cyclohexanon oder Benzaldehyd geschützt sein.
Die Hydroxyschutzgruppen können nach den dem Fachmann bekannten Literaturmethoden, z. B. durch Hydrogenolyse, Säurebehandlung der Ether und Ketale, Alkalibehandlung der Ester oder Behandlung der Silylschutgruppen mit Fluorid freigesetzt werden (siehe z. B. Protective Groups in Organic Syntheses, secon Edition, T.W. Greene and P.G. M. Wuts, John Wiley & Sons, Inc., New Yourk, 1991).

Die $NH_2$-Gruppen lassen sich in vielfältiger Weise schützen und wieder freilegen. Das N-Trifluoracetylderivat wird durch Kalium- oder Natriumcarbonat in Wasser [H. Newman, J. Org. Chem., 30:287 (1965), M. A. Schwartz et al., J. Am. Chem. Soc., 95 G12 (1973)] oder einfach durch Ammoniaklösung gespalten [M. Imazama u. F. Eckstein, J. Org. Chem., 44:2039 (1979)]. Ebenfalls milde zu spalten ist das tert-Butyloxycarbonylderivat: es genügt Rühren mit Trifluoressigsäure [B. F. Lundt et al., J. Org. Chem., 43:2285 (1978)]. Sehr groß ist die Gruppe der hydrogenolytisch oder reduzierend zu spaltenden $NH_2$-Schutzgruppen: Die N-Benzylgruppe ist bequem mit Wasserstoff/Pd-C zu spalten [W.H. Hartung und R. Simonoff, Org. Reactions VII, 263 (1953)], was auch für die Tritylgruppe [L. Zervas, et al., J. Am. Chem. Soc., 78:1359 (1956)] und die Benzyloxycarbonylgruppe gilt [M.Bergmann u. L. Zervas Ber. 65:1192 (1932)]. Von den Silylderivaten werden die leicht spaltbaren tert-Butyldiphenylsilylverbindungen [L. E. Overman et al., Tetrahedron Lett., 27:4391 (1986)], wie auch die 2-(Trimethylsilyl)-ethyl carbamate [L. Grehn et al., Angew. Chem. Int. Ed. Engl., 23:296 (1983)] und die 2-Trimethylsilylethansulfonamide [R.S. Garigipati u. S.M. Weinreb, J. Org. Chem., 53: 4134 (1988)] verwendet, die mit Fluoridionen gespalten werden können. Besonders leicht spaltbar ist das 9-Fluorenylmethyl-carbamat: Die Spaltung erfolgt mit Aminen wie Piperidin, Morpholin, 4-Dimethylaminopyridin, aber auch mit Tetrabutylammoniumfluorid [L. A. Corpino et al., J. Org. Chem., 55:1673 (1990); M. Ueki u. M. Amemiya, Tetrahedron Lett., 28:6617(1987)].

Die Isolierung des erhaltenen Endproduktes der allgemeinen Formel I (Amin oder Carbonsäure) erfolgt ebenfalls nach üblichen und dem Fachmann gut bekannten Methoden.

So wird beispielsweise im Fall der Säureschutzgruppe das Lösungsmittel aus der Hydrolysereaktion abgedampft und der Rückstand in einem aprotischen Lösungsmittel aufgenommen. Durch Ansäuern mit einer wäßrigen Säurelösung wird der pH auf ca. 2-4 eingestellt und danach die organische Phase abgetrennt. Mittels Kristallisation oder Chromatographie kann nun das perbenzylierte 1-O-Glycosid gewonnen werden.

Gewünschtenfalls können die erhaltenen Verbindungen der allgemeinen Formel I auch in üblicher Weise in ihre Salze überführt werden.

Die Ausbeuten der Verbindungen der allgemeinen Formel I, die mit dem erfindungsgemäßen Verfahren erzielt

werden können, sind gut. Sie liegen für bekannte Verbindungen, bei denen ein Vergleich mit dem Stand der Technik möglich ist, über den Ausbeuten des Standes der Technik. So wird beispielsweise für 1-O-Essigsäure von perbenzylierter Glucose eine Gesamtausbeute von 59% in Angew. Chem. 1998, 110 (24), S. 3634 mit dem dort genannten Verfahren beschrieben, während erfindungsgemäß die Ausbeute für diese Verbindung über 2 Stufen 82% beträgt (vgl. Beispiel 7 der vorliegenden Anmeldung). Auch die Herstellung der Verbindung des Beispiels 12 der vorliegenden Anmeldung wird in dieser Publikation beschrieben. Während die Ausbeute an dieser Verbindung erfindungsgemäß 78% über 2 Stufen beträgt, werden mit dem in der Publikation beschriebenen Verfahren lediglich 45% erreicht.

Neben den hohen Ausbeuten bietet das erfindungsgemäße Verfahren auch den Vorteil, daß es von kostengünstigen Startmaterialien ausgeht, ein scale-up des Prozesses ermöglicht und eine leichte Isolierung der Endprodukte erlaubt.

Die Ausgangsmaterialien sind Kaufware oder aus käuflichen Vorstufen leicht erhältlich. So ist bei der Fluka AG, Buchs, Schweiz, die Tetra-2,3,4,6-O-benzyl-D-glucopyranose erhältlich. Bei Fluka sind auch Methyl-D-mannopyranosid und Methyl-D-galactopyranosid Katalogware. Durch Benzylierung und Spaltung des Glycosids sind 2,3,4,6-Tetra-O-benzyl-D-mannose bzw. -galactose erhältlich.
Über die Sequenz-Methylglycosid-Perbenzyl-methylglycosid-Perbenzyl-1-OH-saccharid lassen sich die Perbenzyl-1-OH-Derivate der Pentosen (Ribose, Arabinose), Hexosen und Desoxyhexosen (Rhamnose, Fucose) gewinnen.

Die erfindungsgemäß hergestellten Verbindungen sind wertvolle Zwischenprodukte in der Synthesechemie. So können sie beispielsweise zum Aufbau von Kohlenhydratdendrimeren, zur Synthese von NMR-Kontrastmitteln und zur Einführung von Zuckerresten in Pharmaka Verwendung finden.

Das erfindungsgemäße Verfahren soll nachfolgend an Ausführungsbeispielen näher erläutert werden.

**Beispiel 1**

2,3,4,6-Tetra-O-benzyl-1-O-carboxymethyl-mannopyranose

Eine Mischung aus 54,1 g (100 mmol) 2,3,4,6-Tetra-O-benzylmannopyranose, 1,70 g (5 mmol) Tetrabutylammoniumhydragensulfat und 33,7 g (600 mmol) feingepulvertes Kaliumhydroxid in 350 ml Diethoxymethan wird auf 0°C abgekühlt. Bei 0°C tropft man 29,3 g (150 mmol) Bromessigsäure tert.butylester über 10 Minuten unter starkem Rühren zu. Man rührt eine Stunde bei 0°C. Man gibt 250 ml MTB (Methyl-tert.butylether) zu, filtriert vom Feststoff ab und dampft das Filtrat im Vakuum zur Trockne ein. Der Rückstand wird in 500 ml Ethanol aufgenommen. Man gibt 40 ml 50 %ige aqu. Natronlauge zu und kocht 0,5 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/nHexan/Ethanol/Essigsäure = 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
50,9 g (85 % d. Th., über 2 Stufen) eines farblosen, zähen Öls

| Elementaranalyse: | | |
|---|---|---|
| ber. | C 72,22 | H 6,40 |
| gef. | C 72,38 | H 6,55 |

**Beispiel 2**

2,3,4,6-Tetra-O-benzyl-1-O-carboxymethyl-mannopyranose

Eine Mischung aus 54,1 g (100 mmol) 2,3,4,6-Tetra-O-benzylmannopyranose, 1,7 g (5 mmol) Tetrabutylammoniumhydrogensulfat und 24 g (600 mmol) feingepulvertes Natriumhydroxid in 350 ml 1,2-Dimethoxyethan wird auf 0°C abgekühlt. Bei 0°C tropft man 29,3 g (150 mmol) Bromessigsäureethylester über 10 Minuten unter starkem Rühren zu. Man rührt eine Stunde bei 0°C. Man gibt 250 ml (Diethoxymethan) zu, filtriert vom Feststoff ab und dampft das Filtrat im Vakuum zur Trockne ein. Der Rückstand wird in 500 ml Ethanol/50 ml Wasser aufgenommen. Man gibt 60 ml 50 %ige aqu. Natronlauge zu und kocht 4 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in

300 ml Wasser, 500 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n=Hexan/ Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
48,5 g (81 % d. Th., über 2 Stufen) eines farblosen, zähen Öls

| Elementaranalyse: | | |
|---|---|---|
| ber. | C 72,22 | H 6,40 |
| gef. | C 72,41 | H 6,61 |

**Beispiel 3**

2,3,4,6-Tetra-O-benzyl-1-O-carboxymethyl-mannopyranose

Eine Mischung aus 54,1 g (100 mmol) 2,3,4,6-Tetra-O-benzylmannopyranose, 0,55 g (5 mmol) Tetramethylammoniumchlorid und 33,7 g (600 mmol) feingepulvertes Kaliumhydroxid in 350 ml Diethoxymethan wird auf 10°C abgekühlt. Bei 10°C tropft man 35,7 g (160 mmol) 6-Bromhexansäureethylester über 10 Minuten unter starkem Rühren zu. Man rührt 2 Stunden bei 10°C. Man gibt 250 ml MTB (Methyl-tert.Butylether) zu, filtriert vom Feststoff ab und dampft das Filtrat im Vakuum zur Trockne ein. Der Rückstand wird in 500 ml Ethanol/50 ml Wasser aufgenommen. Man gibt 60 ml 50 %ige aqu. Natronlauge zu und kocht 4 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n= Hexan/ Ethanol/ Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
51,7 g (79 % d. Th., über 2 Stufen) eines farblosen Feststoffs

| Elementaranalyse: | | |
|---|---|---|
| ber. | C 73,37 | H 7,08 |
| gef. | C 73,50 | H 7,27 |

**Beispiel 4**

2,3,4,6-Tetra-O-benzyl-1-O-(1-phenyl-1-carboxy-eth-2-yl)-manopyranose

Eine Mischung aus 54,1 g (100 mmol) 2,3,4,6-Tetra-O-benzylmannopyranose, 1,39 g (5 mmol) Tetrabutylammoniumchlorid und 35,8 g (110 mmol) feingepulvertes Cäsiumcarbonat in 400 ml Diethoxymethan wird auf 0°C abgekühlt. Bei 0°C tropft man 38,6 g (150 mmol) 2-Phenyl-3-brompropionsäure-ethylester, gelöst in 30 ml Diethoxymethan über 10 Minuten unter starkem Rühren zu. Man rührt eine Stunde bei 0°C. Man gibt 250 ml MTB (Methyltert. Butylether) zu, filtriert vom Feststoff ab und dampft das Filtrat im Vakuum zur Trockne ein. Der Rückstand wird in 500 ml Ethanol/50 ml Wasser aufgenommen. Man gibt 60 ml 50 %ige aqu. Natronlauge zu und kocht 4 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n= Hexan/ Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt.

Anschließend wird die organische Phase abgetrennt und im Vakuum zur Trockne eingedampft.
Ausbeute:
54,4 g (79 % d. Th., über 2 Stufen) eines farblosen Feststoffs

| Elementaranalyse: | | |
|---|---|---|
| ber. | C 74,98 | H 6,44 |
| gef. | C 75,11 | H 6,58 |

**Beispiel 5**

2,3,4,6-Tetra-O-benzyl-1-O-carboxymethyl-mannopyranose

Eine Mischung aus 54,1 g (100 mmol) 2,3,4,6-Tetra-O-benzylmannopyranose, 1,39 g (5 mmol) Tetrabutylammoniumchlorid und 15,2 g (110 mmol) wasserfreiem Kaliumcarbonat in 500 ml Diethoxymethan wird auf 0°C abgekühlt. Bei 0°C tropft man 30,12 g (200 mmol Chloressigsäure-tert.-butylester über 20 Minuten unter starkem Rühren zu. Man rührt eine Stunde bei 10°C. Man gibt 250 ml Methyl-tert.Butylether zu, trennt die organische Phase ab, extrahiert die wässrige Phase 2 mal mit 250 ml Wasser. Das Lösungsmittel der vereinigten organischen Phasen wird mit Wasser gewaschen, über Natriumsulfat getrocknet, abfiltriert und im Vakuum abdestilliert.Der Rückstand wird in 500 ml Ethanol aufgenommen. Man gibt 40 ml 50 %ige aqu. Natronlauge zu und kocht 0,5 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n= Hexan/Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
41,1 g (82 % d. Th., über 2 Stufen) eines farblosen, zähen Öls

| Elementaranalyse: | | |
|---|---|---|
| ber. | C 72,22 | H 6,40 |
| gef. | C 72,01 | H 6,63 |

**Beispiel 6**

2,3,4,6-Tetra-O-benzyl-1-O-carboxymethyl-glucopyranose

Eine Mischung aus 54,1 g (100 mmol) 2,3,4,6-Tetra-O-benzylglucopyranose 1,39 g (5 mmol) Tetrabutylammoniumchlorid und 15,22 g (100 mmol) DBU in 300 ml Diethoxymethan wird auf 0°C abgekühlt. Bei 0°C tropft man 78 g (150 mmol) 5-Tosyloxypentancarbonsäure-tert.butylester, gelöst in 40 ml Tetrahydrofuran über 30 Minuten unter starkem Rühren zu. Man rührt 3 Stunden bei 0°C. Man gibt 300 ml MTB (Methyltert.Butylether) zu, filtriert vom Feststoff ab und dampft das Filtrat im Vakuum zur Trockne ein. Der Rückstand wird in 500 ml Methanol aufgenommen. Man gibt 50 ml 50 %ige aqu. Natronlauge zu und kocht 1 Stunde unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Dichlormethan nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n= Hexan/ Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
50 g (78 % d. Th., über 2 Stufen) eines farblosen Feststoffs

| Elementaranalyse: | | |
|---|---|---|
| ber. | C 73,10 | H 6,92 |
| gef. | C 73,21 | H 7,09 |

**Beispiel 7**

2,3,4,6-Tetra-O-benzyl-1-O-carboxymethyl-glucopyranose

Eine Mischung aus 54,1 g (100 mmol) 2,3,4,6-Tetra-O-benzylglucopyranose, 1,39 g (5 mmol) Tetrabutylammoniumchlorid und 11,22 g Kalium t butoxid (100 mmol) in 500 ml Diethoxymethan wird auf 0°C abgekühlt. Bei 0°C tropft man 29,3 g (150 mmol Bromessigsäure-tert.-butylester über 20 Minuten unter starkem Rühren zu. Man rührt 0,5 Stunden bei 0°C. Man gibt 250 Toluol zu, trennt die organische Phase ab, extrahiert die wässrige Phase 2 mal mit 150 ml Toluol. Das Lösungsmittel der vereinigten organischen Phasen wird mit Wasser gewaschen, über Natriumsulfat getrocknet, vom Trocknungsmittel abfiltriert und im Vakuum abdestilliert. Der Rückstand wird in 400 ml Methanol aufgenommen. Man gibt 50 ml 50 %ige aqu. Natronlauge zu und kocht 0,5 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Dichlormethan auf und stellt unter Rühren den pH-Wert der wässrigen Phase auf pH 2 (10 %ige aqu. Salzsäure). Die organische Phase wird abgetrennt, die wässrige Phase noch einmal mit 200 ml Dichlormethan nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n-Hexan/ Ethanol/ Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
49,1 g (82 % d. Th., über 2 Stufen) eines farblosen, zähen Öls

| Elementaranalyse: | | |
|---|---|---|
| ber. | C 72,22 | H 6,40 |
| gef. | C 72,09 | H 6,59 |

**Beispiel 8**

2,3,4,6-Tetra-O-benzyl-1-O-carboxymethyl-glucopyranose

Eine Mischung aus 54,1 g (100 mmol) 2,3,4,6-Tetra-O-benzylglucopyranose, 0,55 g (5 mmol) Tetramethylammoniumchlorid und 12,42 g (100 mmol) DBN in 350 ml Diethoxymethan wird auf 0°C abgekühlt. Bei 0°C tropft man 44 g (150 mmol) 11-Bromundecansäure-ethylester, gelöst in 50 ml Benzol über 30 Minuten unter starkem Rühren zu. Man rührt 2 Stunden bei 20°C. Man gibt 250 ml Methyl-tert.Butylether zu, filtriert vom Feststoff ab und dampft das Filtrat im Vakuum zur Trockne ein. Der Rückstand wird in 500 ml Ethanol/50 ml Wasser aufgenommen. Man gibt 60 ml 50 %ige aqu. Natronlauge zu und kocht 5 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Dichlormethan auf und stellt unter Rühren den pH-Wert der wässrigen Phase auf pH 2 (10 %ige aqu. Salzsäure). Die organische Phase wird abgetrennt, die wässrige Phase noch einmal mit 200 ml Dichlormethan nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n-Hexan/ Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
58,4 g (78 % d. Th., über 2 Stufen) eines farblosen Feststoffs

| Elementaranalyse: | | |
|---|---|---|
| ber. | C 75,37 | H 7,54 |
| gef. | C 75,52 | H 7,73 |

**Beispiel 9**

2,3,4,6-Tetra-O-benzyl-1-O-carboxymethyl-galactopyranose

Eine Mischung aus 54,1 g (100 mmol) 2,3,4,6-Tetra-O-benzylmannopyranose, 1,39 g (5 mmol) Tetrabutylammoniumchlorid und 9,62 g (100 mmol) Natrium t butoxid in 350 ml Diethoxymethan wird auf 0°C abgekühlt. Bei 0°C tropft man 30,12 g (200 mmol Chloressigsäure-tert.-butylester über 20 Minuten unter starkem Rühren zu. Man rührt eine Stunde bei 10°C. Man gibt 250 ml Methyl-tert.Butylether zu, trennt die organische Phase ab, extrahiert die wässrige Phase 2 mal mit 250 ml Wasser. Das Lösungsmittel der vereinigten organischen Phasen wird über Natriumsulfat getrocknet,vom trocknungsmittel abfiltriert und im Vakuum abdestilliert. Der Rückstand wird in 500 ml Ethanol aufgenommen. Man gibt 40 ml 50 %ige aqu. Natronlauge zu und kocht 0,5 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n= Hexan/Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
41,1 g (82 % d. Th., über 2 Stufen) eines farblosen, zähen Öls

| Elementaranalyse: | | |
|---|---|---|
| ber. | C 72,22 | H 6,40 |
| gef. | C 72,03 | H 6,63 |

**Beispiel 10**

2,3,4,6-Tetra-O-benzyl-1-O-[1-(4-carboxy)-phenyl-prop-3-ylgalactopyranose

Eine Mischung aus 54,1 g (100 mmol) 2,3,4,6-Tetra-O-benzylgalactopyranose, 1,39 g (5 mmol) Tetrabutylammoniumchlorid und 24 g (600 mmol) feingepulvertes Natriumhydroxid in 300 ml Diethoxymethan wird auf 10°C abgekühlt. Bei 10°C tropft man 43 g (150 mmol) 4-(3-Methansulfonyloxy-propyl)-benzoesäureethylester, gelöst in 50 ml Tetrahydrofuran über 30 Minuten unter starkem Rühren zu. Man rührt 2 Stunden bei 10°C. Man gibt 300 ml MTB (Methyl-tert.Butylether) zu, filtriert vom Feststoff ab und dampft das Filtrat im Vakuum zur Trockne ein. Der Rückstand wird in 500 ml Methanol/50 ml Wasser aufgenommen. Man gibt 60 ml 50 %ige aqu. Natronlauge zu und kocht 5 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n=Hexan/ Ethanol/Essigsäure= 20:5:3:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
54,1 g (77 % d. Th., über 2 Stufen) eines farblosen Feststoffs

| Elementaranalyse: | | |
|---|---|---|
| ber. | C 75,19 | H 6,60 |
| gef. | C 75,02 | H 6,79 |

**Beispiel 11**

2,3,5-Tri-O-benzyl-1-O-carboxymethyl-ribofuranose

Eine Mischung aus 42,1 g (100 mmol) 2,3,5-Tri-O-ribofuranose, 1,39 g (5 mmol) Tetrabutylammoniumchlorid

in 350 ml Diethoxymethan und 200 ml 50 %ige aqu. Natron-Lauge wird auf 0°C abgekühlt. Bei 0°C tropft man 29,3 g (150 mmol Bromessigsäure-tert.-butylester über 20 Minuten unter starkem Rühren zu. Man rührt eine Stunde bei 0°C. Man gibt 250 ml Methyl-tert.butylether zu, trennt die organische Phase ab, extrahiert die wässrige Phase 2 mal mit 200 ml Methyltert.butylether. Das Lösungsmittel der vereinigten organischen Phasen wird über Natriumsulfat getrocknetn,vom Trocknungsmittel abfiltriert und im Vakuum abdestilliert und der Rückstand in 500 ml Ethanol aufgenommen. Man gibt 50 ml 50 %ige aqu. Natronlauge zu und kocht 0,5 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 500 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n Hexan/Ethanol/Essigsäure= 20:5:3:0,5). Die produktenthaltenden Fraktionen werden eingedampft, in 200 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
39,2 g (82 % d. Th., über 2 Stufen) eines farblosen, zähen Öls

| Elementaranalyse: | | |
|---|---|---|
| ber. | C 70,28 | H 6,32 |
| gef. | C 70,11 | H 6,51 |

**Beispiel 12**

2,3,5-Tri-O-benzyl-1-O-(1-amino-eth-2-yl)-ribofuranose

Eine Mischung aus 42,1 g (100 mmol) 2,3,5-Tri-O-benzylribofuranose, 3,40 g (10 mmol) Tetrabutylammoniumhydrogensulfat und 33,7 g (600 mmol) feingepulvertes Kaliumhydroxid in 350 ml Diethoxymethan wird auf 10°C abgekühlt. Bei 10°C tropft man 38,1 g (150 mmol)N-(2-Bromethyl)-phthalimid, gelöst in 100 ml Benzol über 40 Minuten unter starkem Rühren zu.. Man rührt 3 Stunden bei 10°C. Man gibt 300 ml Benzol zu, filtriert vom Feststoff ab und dampft das Filtrat im Vakuum zur Trockne ein. Der Filtratrückstand wird in 500 ml Ethanol gelöst, 25,03 g Hydrazinhydrat (500 mmol) zugegeben und 6 Stunden unter Rückfluß erhitzt. Man läßt auf 0°C abkühlen, filtriert vom ausgefallenen Niederschlag ab und dampft das Filtrat im Vakuum zu Trockne ein. Der Rückstand wird in 400 ml Dichlormethan gelöst, diese Lösung 2 mal mit 5 %iger aqu. Natronlauge, anschließend einmal mit Wasser (jeweils 300 ml) gewaschen. Die organische Phase wird im Vakuum zur Trockne eingedampft der Rückstand an Kieselgel chromatographiert (Laufmittel: Dichlormethan/Ethanol/Triethylamin= 20:2:0,1).
Ausbeute:
36,2 g (78 % d. Th., über 2 Stufen) eines farblosen Feststoffs

| Elementaranalyse: | | | |
|---|---|---|---|
| ber. | C 72,55 | H 7,17 | N 3,02 |
| gef. | C 72,39 | H 7,38 | N 2,87 |

**Beispiel 13**

2,3,4,6-Tetra-O-benzyl-1-O-(1-amino-prop-3-yl)-galactopyranose

Eine Mischung aus 42,1 g (100 mmol) 2,3,4,6-Tetra-O-benzylgalactopyranose, 1,7 g (5 mmol) Tetrabutylammoniumhydrogensulfat und 33,7 g (600 mmol) feingepulvertes Kaliumhydroxid in 350 ml Diethoxymethan wird auf 10°C abgekühlt. Bei 10°C tropft man 40,2 g (150 mmol) N-(3-Brompropyl)-phthalimid, gelöst in 100 ml 1,2-Dimethoxyethan über 40 Minuten unter starkem Rühren zu. Man rührt 3 Stunden bei 10°C. Man gibt 300 ml Benzol zu, filtriert vom Feststoff ab und dampft das Filtrat im Vakuum zur Trockne ein. Der Filtratrückstand wird in 500 ml Ethanol gelöst, 25,03 ml Hydrazinhydrat (500 mmol) zugegeben und 6 Stunden unter Rückfluß erhitzt. Man läßt auf 0°C abkühlen, filtriert vom ausgefallenen Niederschlag ab und dampft das Filtrat im Vakuum zu Trockne ein. Der Rückstand wird in 400 ml Dichlormethan gelöst, diese Lösung 2 mal mit 5 %iger aqu. Natronlauge, anschließend einmal mit Wasser (jeweils 300 ml) gewaschen. Die organische Phase wird im Vakuum zur Trockne eingedampft, der Rückstand an Kieselgel chromatographiert (Laufmittel: Dichlormethan/Ethanol/Triethylamin= 20:2:0,1).

Ausbeute:
46 g (77 % d. Th. über 2 Stufen) eines farblosen Feststoffs

| Elementaranalyse: | | | |
|---|---|---|---|
| ber. | C 74,35 | H 7,25 | N 2,34 |
| gef. | C 74,24 | H 7,41 | N 2,27 |

**Beispiel 14**

2,3,4,6-Tetra-O-benzyl-1-O-(1-amino-hex-6-yl)-mannopyranose

Eine Mischung aus 54,1 g (100 mmol) 2,3,4,6-Tetra-O-benzylmannopyranose, 1,39 g (5 mmol) Tetrabutylammoniumchlorid und 35,84 g (110 mmol) Cäsiumcarbonat in 500 ml Diethoxymethan wird auf 0°C abgekühlt. Bei 0°C tropft man 60,3 g (150 mmol 6-Brom-hexylamin-N-(9-fluorenylmethoxy-carbonyl) über 30 Minuten unter starkem Rühren zu. Man rührt eine Stunde bei 0°C. Man gibt 300 ml Dichlormethan zu, trennt die organische Phase ab, extrahiert die wässrige Phase 2 mal mit 200 ml Dichlormethan. Das Lösungsmittel der vereinigten organischen Phasen wird im Vakuum abdestilliert. Der Rückstand wird in 250 ml Ethanol aufgenommen und 100 g (1,17 mol) Piperidin zugegeben. Man rührt 5 Stunden bei 40 °C. Die Lösung wird zur Trockne eingedampft und der Rückstand an Kieselgel chromatographiert (Laufmittel: Dichlormethan/ Ethanol/ Triethylamin= 20:2:0,1).
Ausbeute:
41,1 g (79 % d. Th. über 2 Stufen) eines farblosen Feststoffs

| Elementaranalyse: | | | |
|---|---|---|---|
| ber. | C 69,33 | H 9,50 | N 2,70 |
| gef. | C 69,44 | H 9,68 | N 2,54 |

**Beispiel 15**

2,3,4-Tri-O-benzyl-6-desoxy-1-O-(1-amino-but-4-yl)-fucopyranose

Eine Mischung aus 43,5 g (100 mmol) 2,3,4-Tri-O-benzyl-6-desoxy-fucopyranose, 1,7 g (5 mmol) Tetrabutylammoniumhydrogensulfat in 350 ml Diethoxymethan und 200 ml 60%ige aqu. Natronlauge wird auf 0°C abgekühlt. Bei 10°C tropft man 47,4 g (150 mmol) 2-(Trimethylsilyl)-ethylsulfonsäure-N-(4-brombutyl)-amid, gelöst in 100 ml Dichlormethan über 30 Minuten unter starkem Rühren zu. Man rührt 2 Stunden bei 10°C. Man gibt 600 ml Dichlormethan zu, trennt die organische Phase ab, extrahiert die wässrige Phase 2 mal mit 200 ml Dichlormethan. Die vereinigten organischen Phasen werden über Natriumsulfat getrocknet. Man filtriert vom Trocknungsmittel und und destilliert das Lösungsmittel im Vakuum ab. Der Rückstand wird in 350 ml Acetonitril aufgenommen und 52,3 g (200 mmol) Tetrabutylammoniumflorid als Monohydrat zugegeben. Man rührt 3 Stunden bei 50 °C. Die Lösung wird zur Trockne eingeengt und der Rückstand an Kieselgel chromatographiert (Laufmittel: Dichlormethan/ Ethanol/ Triethylamin= 20:2:0,1).
Ausbeute:
39,4 g (78 % d. Th. über 2 Stufen) eines farblosen Feststoffs

| Elementaranalyse: | | | |
|---|---|---|---|
| ber. | C 73,64 | H 7,77 | N 2,77 |
| gef. | C 73,53 | H 7,91 | N 2,65 |

**Beispiel 16**

2,3,4,6-Tetra-O-benzyl-1-0-(3,6,9,12,15-pentaoxa-1-carboxyhexadec-16-yl)-glucopyranose

Eine Mischung aus 54,1 g (100 mmol) 2,3,4,6-Tetra-O-benzylglucopyranose, 1,39 g (5 mmol) Tetrabutylammoniumchlorid und 24 g (600 mmol) feingepulvertes Natriumhydroxid in 350 ml Diethoxymethan wird auf 0°C abgekühlt. Bei 0°C tropft man 64,3 g (130 mmol) 17-Tosyloxy-3,6,9,12,15-pentaoxaheptadecansäureethylester, gelöst in 100 ml Tetrahydrofuran über 50 Minuten unter starkem Rühren zu. Man rührt 3 Stunden bei 0°C. Man gibt 300 ml

Dichlormethan zu, filtriert vom Feststoff ab und dampft das Filtrat im Vakuum zur Trockne ein. Der Rückstand wird in 500 ml Ethanol/100 ml Wasser aufgenommen. Man gibt 60 ml 60 %ige aqu. Natronlauge zu und kocht 5 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 400 ml Essigsäureethylester auf und stellt unter Rühren den pH-Wert der wässrigen Phase auf pH 2 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/n Hexan/Ethanol/Essigsäure= 20:8:5:0,5). Die produktenthaltenden Fraktionen werden eingedampft, in 400 ml Essigsäureethylester gelöst und 3 mal mit 200 ml Wasser ausgeschüttelt. Anschließend wird die organische Phase abgetrennt und im Vakuum zur Trockne eingedampft.
Ausbeute:
64,3 g (77 % d. Th., über 2 Stufen) eines farblosen Öls

| Elementaranalyse: | | |
|---|---|---|
| ber. | C 66,17 | H 7,00 |
| gef. | C 66,03 | H 7,19 |

**Beispiel 17**

2,3,4,6-Tetra-O-benzyl-1-0-(1-hydroxy-eth-2-yl)-mannopyranose

Eine Mischung aus 54,1 g (100 mmol) 2,3,4,6-Tetra-O-benzylmannopyranose, 1,7 g (5 mmol) Tetrabutylammoniumhydrogensulfat und 33,7 g (600 mmol) feingepulvertes Kaliumhydroxid in 350 ml Diethoxymethan wird auf 0°C abgekühlt. Bei 0°C tropft man 31,4 g(150 mmol) 2,2-Dimethyl-propionsäure-2-bromethylester, über 30 Minuten unter starkem Rühren zu. Man rührt 2 Stunden bei 0°C. Man gibt 300 ml Benzol zu, filtriert vom Feststoff ab und dampft das Filtrat im Vakuum zur Trockne ein. Der Rückstand wird in 500 ml Ethanol/100 ml Wasser aufgenommen. Man gibt 100 ml 50 %ige aqu. Kalilauge zu und kocht 8 Stunden unter Rückfluß. Es wird auf 0°C abgekühlt, mit 10 %iger aqu. Salzsäure auf pH 8 gestellt und anschließend das Lösungsmittel abdestilliert (Vakuum). Man nimmt den Rückstand in 300 ml Wasser, 400 ml Essigsäureethylester auf und stellt unter Rühren den pH-Wert der wässrigen Phase auf pH 5 (10 %ige aqu. Salzsäure). Die organische Phase wird abgetrennt, die wässrige Phase noch einmal mit 200 ml Essigsäureethylester nachextrahiert. Die vereinigten organischen Phasen werden über Magnesiumsulfat getrocknet, das Lösungsmittel im Vakuum abdestilliert und der Rückstand an Kieselgel chromatographiert (Laufmittel: Dichlormethan/nHexan/ Ethanol= 20:8:2). Die produktenthaltenden Fraktionen werden eingedampft.
Ausbeute:
45,6 g (78 % d. Th., über 2 Stufen) eines farblosen, zähen Öls

| Elementaranalyse: | | |
|---|---|---|
| ber. | C 73,95 | H 6,90 |
| gef. | C 73,84 | H 7,03 |

**Beispiel 18**

2,3,4,6-Tetra-O-benzyl-1-O-carboxymethyl-mannopyranose

Eine Mischung aus 500,0 g (924,2 mmol) 2,3,4,6-Tetra-O-benzylmannopyranose und 50.00g (147,1 mmol) Tetrabutylammoniumhydrogensulfat in 2500 ml Diethoxymethan wird auf eine Temperatur von0° C abgekühlt. Man gibt dann 121,77 g (217,0 mmol) feingepulvertes Kaliumhydroxid dazu und rührt 10 Minuten nach. Bei einer Temperatur zwischen 0° und 5° C tropft man innerhalb von 30 Minuten 180,39 g (1608 mmol) Bromessigsäure tert.butylester unter starkem Rühren zu. Und rührt zwei Stunden bei 0°C nach. Man läßt dann innerhalb von zwei Stunden auf Raumtemperatur kommen, gibt 500 ml absolutes Ethanol (mit Cyclohexan vergällt) dazu und destilliert bei einer Badtemperatur von 105° C das Lösungsmittel weitgehend ab. Nun gibt man 1000 ml Ethanol (abs. mit Cyclohexan vergällt) dazu und destillieert erneut bei 105° C Badtemperatur das Lösungsmittel weitgehend ab. Darauf werden erneut 1000 ml Ethanol (abs., mit Cclohexan vergällt) zugegeben sowie 60,89 g (1085 mmol) Kaliumhydroxid (als Pulver) sowie 1000 ml vollentsalztes Wasser. Man erhitzt 7 Stunden am Rückfluss und engt dann zu einem noch rührbaren Öl ein. Nach dem Abkühlen auf Raumtemperatur wird der Rückstand 3 mal mit je 500 ml Hexan extrahiert. Die wässrige Phase wird mit 500 ml MTB versetzt und unter starkem Rühren mit 37% Salzsäure auf einen pH-Wert von 2 eingestellt. Die wässrige

Phase wird dann zweimal mit je 1000 ml MTB extrahiert. Die organischen Phasen werden vereinigt, getrocknet und am Rotationsverdampfer bei einer Badtemperatur von 50° C und einem Druck von 70 mbar zur Trockne eingeengt. Der Rückstand wird an 1000 g Kieselgel 60 (Korngrösse 40-63 µm) chromatographiert. Als Elutionsmittel werden 2700 ml nHexan, 5400 ml Dichlormethan sowie 1500 ml Methanol.verwendet. Die produktenthaltenden Fraktionen werden vereinigt und im Vakuum zur Trockene eingedampft.
Ausbeute:
246,33 g (52,6% d. Th., über 2 Stufen) eines farblosen, zähen Öls

| Elementaranalyse: | | |
|---|---|---|
| ber. | C 72,22 | H 6,40 |
| gef. | C 72,40 | H 6,54 |

**Patentansprüche**

**1.** Verfahren zur Herstellung von perbenzylierten 1-O-Glycosiden der allgemeinen Formel I

$$\underset{\textstyle (OR)_n}{\overset{|}{\text{Zucker}^1}} - O - L - X - H \qquad (I),$$

in der
Zucker[1] ein in 1-OH-Position funktionalisiertes Monosaccharid ist,

R Benzyl darstellt,
n 2, 3 oder 4 bedeutet,
X -COO- oder -NH- bedeutet

und

L eine geradkettige, verzweigte, gesättigte oder ungesättigte $C_1$-$C_{30}$-Kohlenstoffkette bedeutet, die gegebenenfalls unterbrochen ist durch 1-10 Sauerstoffatome, 1-3 Schwefelatome, 1-2 Phenylen-, 1-2 Phenylenoxy-, 1-2 Phenylendioxygruppen, einen Thiophen-, Pyrimidin- oder Pyridinrest und/oder gegebenenfalls substituiert ist mit 1-3 Phenyl-, 1-3 Carboxyl-, 1-5 Hydroxy-, 1-5 O-$C_1$-$C_7$alkyl-, 1-3 Aminogruppen, 1-3 $CF_3$-Gruppen oder 1-10 Fluoratomen

oder deren Salzen
**dadurch gekennzeichnet, daß**
ein perbenzylierter 1-OH-Zucker der allgemeinen Formel II

$$\underset{\textstyle (OR)_n}{\overset{|}{\text{Zucker}^1}} - OH \qquad (II),$$

worin Zucker[1], R und n die angegebene Bedeutung haben, mit einem Alkylierungsreagenz der allgemeine Formel (III)

$$Nu - L - X - Sg \qquad (III),$$

worin Nu ein Nucleofug bedeutet, L und X die genannte Bedeutung haben und Sg eine Schutzgruppe darstellt, in dem organischen Lösungsmittel Diethoxymethan in Gegenwart einer Base und gegebenenfalls eines Phasentransfer-Katalysators bei einer Temperatur von 0-50°C umgesetzt wird, anschließend die Schutzgruppe abgespalten wird und das erhaltene Reaktionsprodukt gegebenenfalls in ein Salz überführt wird.

**2.** Verfahren nach Anspruch 1,
**dadurch gekennzeichnet, daß**
als perbenzylierter 1-OH-Zucker der allgemeinen Formel II ein perbenzyliertes Monosaccharid mit 5 bis 6 C-Atomen oder dessen Desoxy-Verbindung eingesetzt wird.

**3.** Verfahren nach Anspruch 1 oder 2,
**dadurch gekennzeichnet, daß**
als perbenzylierter 1-OH-Zucker der allgemeinen Formel II perbenzylierte Glucose, Mannose, Galactose, Ribose, Arabinose, Xylose, Fucose oder Rhamnose eingesetzt wird.

**4.** Verfahren nach einem der Ansprüche 1 bis 3,
**dadurch gekennzeichnet, daß**
als Alkylierungsreagenz der allgemeinen Formel III ein solches eingesetzt wird, in welchem das Nucleofug die Reste -Cl, -Br, -J, -OTs, -OMs, $-OSO_2CF_3$, $-OSO_2C_4F_9$ oder $-OSO_2C_8F_{17}$ bedeutet.

**5.** Verfahren nach einem der Ansprüche 1 bis 4,
**dadurch gekennzeichnet, daß**
als Alkylierungsreagenz der allgemeinen Formel III ein solches eingesetzt wird, in welchem der Rest L

$$\gamma-(CH_2)_n-\delta \quad n=1\text{-}15$$

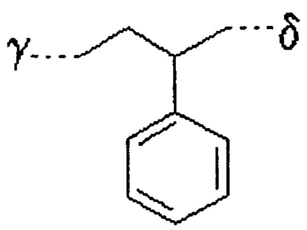

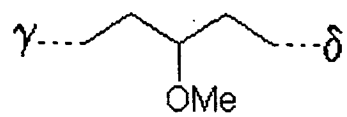

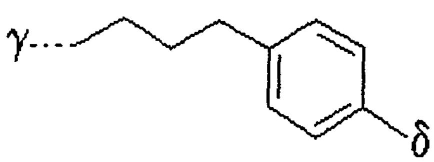

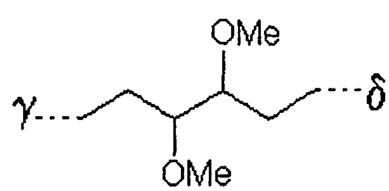

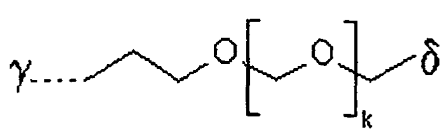

k = 0-9

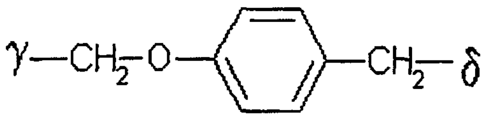

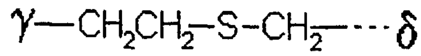

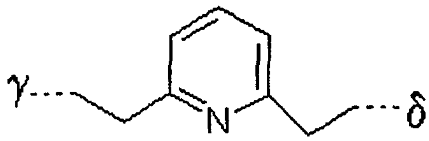

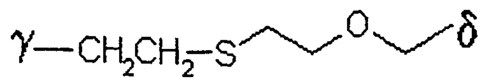

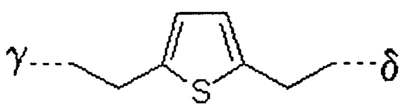

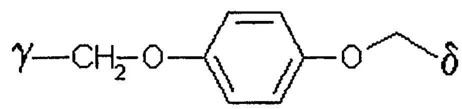

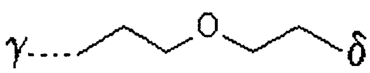

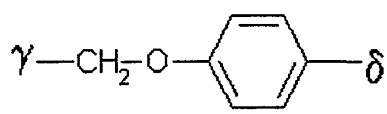

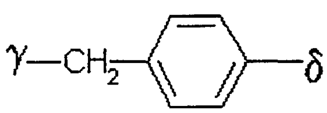

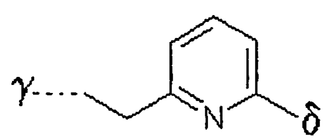

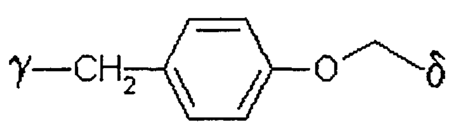

bedeutet,

wobei γ die Verknüpfungsstelle am Zucker bedeutet und δ die Verknüpfungsstelle zum Rest X ist.

**6.** Verfahren nach einem der Ansprüche 1 bis 5,
**dadurch gekennzeichnet, daß**
als Phasentransfer-Katalysator ein quartäres Ammcnium- oder Phosphoniumsalz oder ein Kronenether eingesetzt wird, vorzugsweise ein quartäres Ammoniumsalz.

**7.** Verfahren nach einem der Ansprüche 1 bis 6, **dadurch gekennzeichnet,daß**
als Base Natriumhydroxid, Kaliumhydroxid, Cäsiumcarbonat, Kaliumcarbonat,.1,8-Diazabi-cyclo[5.4.0]undec-7-en, 1,5-Diazabicyclo[2.2.2]octan, Kalium t butoxid und Natriumt butoxid eingesetzt wird.

**8.** Verfahren nach einem der Ansprüche 1 bis 7, **dadurch gekennzeichnet,daß**
die Base in fester oder flüssiger Form oder als 10 - 70% Lösung zugesetzt wird.

## Claims

**1.** Process for the production of perbenzylated 1-O-glycosides of general formula I

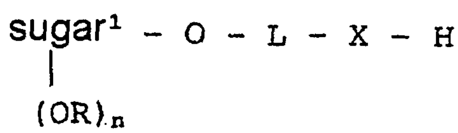

(I),

in which
sugar[1] is a monosaccharide that is functionalized in 1-OH-position,
R represents benzyl,
n means 2, 3 or 4,
X means -COO- or -NH-
and
L means a straight-chain, branched, saturated or unsaturated $C_1$-$C_{30}$ carbon chain, which optionally is interrupted by 1-10 oxygen atoms, 1-3 sulphur atoms, 1-2 phenylene groups, 1-2 phenyleneoxy groups, 1-2 phenylenedioxy groups, a thiophene radical, pyrimidine radical or pyridine radical, and/or optionally is substituted with 1-3 phenyl groups, 1-3 carboxyl groups, 1-5 hydroxy groups, 1-5 O-$C_1$-$C_7$ alkyl groups, 1-3 amino groups, 1-3 $CF_3$ groups or 1-10 fluorine atoms or their salts,
**characterized in that**
a perbenzylated 1-OH-sugar of general formula II

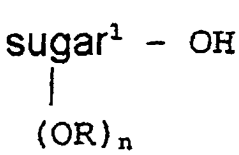

(II),

in which sugar [1], R and n have the indicated meaning, is reacted with an alkylating reagent of general formula (III)

Nu-L-X-Sg (III),

in which Nu means a nucleofuge, L and X have the above-mentioned meaning, and Sg represents a protective

group, in the organic solvent diethoxymethane in the presence of a base and optionally a phase transfer catalyst at a temperature of 0-50°C, then the protective group is cleaved off, and the reaction product that is obtained is optionally converted into a salt.

**2.** Process according to Claim 1, **characterized in that** a perbenzylated monosaccharide with 5 to 6 C-atoms or its deoxy compound is used as a perbenzylated 1-OH-sugar of general formula II.

**3.** Process according to Claim 1 or 2, **characterized in that** perbenzylated glucose, mannose, galactose, ribose, arabinose, xylose, fucose or rhamnose is used as a perbenzylated 1-OH-sugar of general formula II.

**4.** Process according to one of Claims 1 to 3, **characterized in that** as an alkylating reagent of general formula III, one is used in which the nucleofuge means radicals -Cl, -Br, -I, -OTs, -OMs, $-OSO_2CF_3$, $-OSO_2C_4F_9$ or $-OSO_2C_8F_{17}$.

**5.** Process according to one of Claims 1 to 4,
**characterized in that** as an alkylating reagent of general formula III, one is used in which radical L means

$$\gamma—(CH_2)_n—\delta \qquad n=1\text{-}15$$

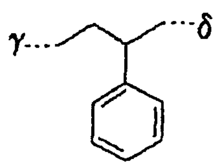

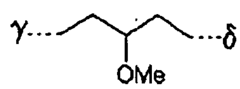

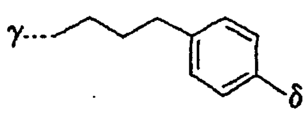

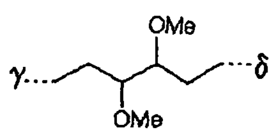

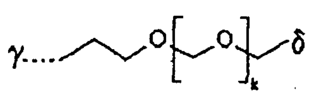

k = 0-9

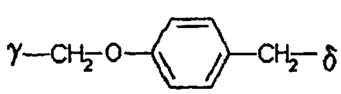

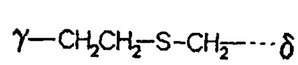

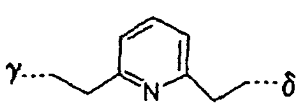

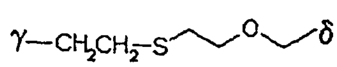

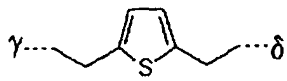
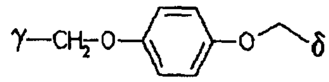

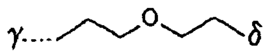
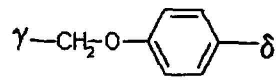

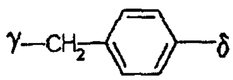
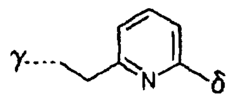

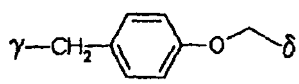
,

whereby γ is the interface site to the sugar, and δ is the interface site to radical X.

6. Process according to one of Claims 1 to 5, **characterized in that** a quaternary ammonium or phosphonium salt or a crown ether, preferably a quaternary ammonium salt, is used as a phase transfer catalyst.

7. Process according to one of claims 1 to 6, wherein sodium hydroxide, potassium hydroxide, caesium carbonate, potassium carbonate, 1,8-diazabicyclo[5.4.0]undec-7-ene, 1,5-diazabicyclo[2.2.2]octane, potassium t-butoxide or sodium t-butoxide is used as a base.

8. Process according to one of Claims 1 to 7, **characterized in that** the base is added in solid or liquid form or as 10-70% solution.

**Revendications**

1. Procédé de préparation de 1-O-glucosides perbenzylés de formule générale I

$$\underset{(OR)_n}{\underset{|}{Sucre^1}} - O - L - X - H \qquad (I),$$

dans laquelle
Sucre[1] représente un monosaccharide fonctionnalisé en position 1-OH,

R représente un benzyle,
n vaut 2, 3 ou 4,
X représente -COO- ou -NH-,
et
L représente une chaîne carbonée en $C_1$-$C_{30}$ linéaire ramifiée, saturée ou insaturée, qui est éventuellement interrompue par 1-10 atomes d'oxygène, 1-3 atomes de soufre, 1-2 groupes phénylène, 1-2 groupes phénylènoxy, 1-2 groupes phénylènedioxy, un radical thiophène, pyrimidine ou pyridine et/ou est éventuellement substituée par 1-3 groupes phényle, 1-3 groupes carboxy, 1-5 groupes hydroxy, 1-5 groupes O-$C_1$-$C_7$-alkyle, 1-3 groupes amino, 1-3 groupes $CF_3$ ou 1-10 atomes de fluor,
ou leurs sels,
**caractérisé en ce que**
un 1-OH-Sucre perbenzylé de formule générale II

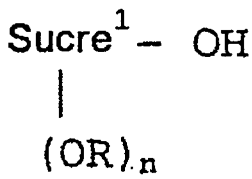

(II),

dans laquelle Sucre [1], R et n présente la signification indiquée,
est mis à réagir avec un réactif d'alkylation de formule générale (III)

Nu - L - X - Sg (III),

dans laquelle Nu représente un nucléofuge, L et X présentent la signification mentionnée et Sg représente un groupe protecteur,
dans le solvant organique diéthoxyméthane en présence d'une base et éventuellement d'un catalyseur à transfert de phase à une température de 0 à 50 °C, puis le groupe protecteur est éliminé et le produit de réaction obtenu est éventuellement transformé en un sel.

2. Procédé selon la revendication 1, **caractérisé en ce que** l'on utilise, en tant que 1-OH-Sucre perbenzylé de formule générale II, un monosaccharide perbenzylé ayant de 5 à 6 atomes de carbone ou son composé désoxy.

3. Procédé selon la revendication 1 ou 2, **caractérisé en ce que** l'on utilise, en tant que 1-OH-Sucre perbenzylé de formule générale II, le glucose, le mannose, le galactose, le ribose, l'arabinose, le xylose, le fucose ou le rhamnose perbenzylés.

4. Procédé selon l'une quelconque des revendications 1 à 3, **caractérisé en ce que** l'on utilise, en tant que réactif d'alkylation de formule générale III, un réactif d'alkylation dans lequel le nucléofuge représente les radicaux -Cl, -Br, -J, -OTs, -OMs, $-OSO_2CF_3$, $-OSO_2C_4F_9$ ou $-OSO_2C_8F_{17}$.

5. Procédé selon l'une quelconque des revendications 1 à 4, **caractérisé en ce que** l'on utilise, en tant que réactif d'alkylation de formule générale III, un réactif d'alkylation dans lequel le radical L représente

$$\gamma—(CH_2)_n—\delta \quad n=1\text{-}15$$

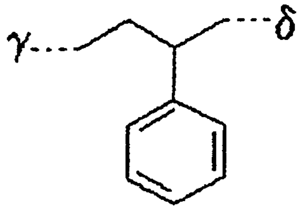

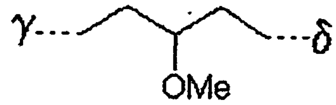

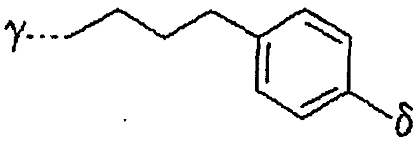

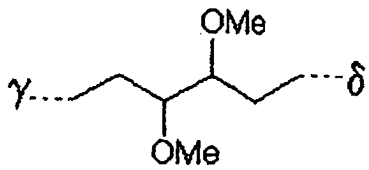

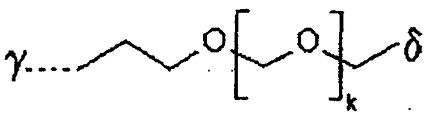

k = 0-9

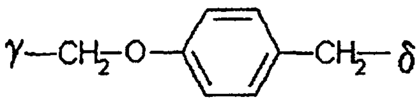

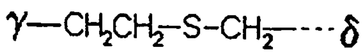

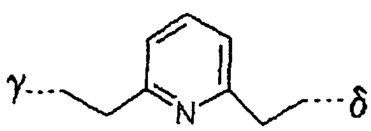

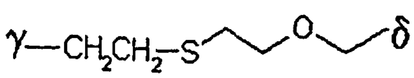

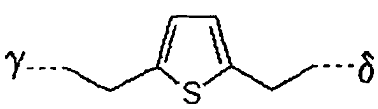

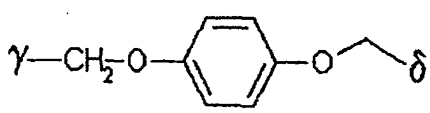

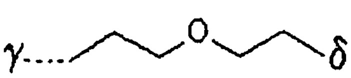

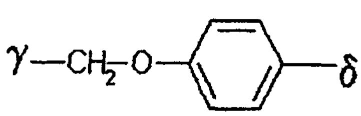

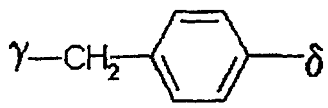

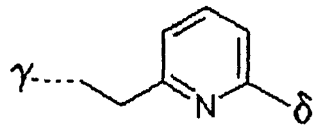

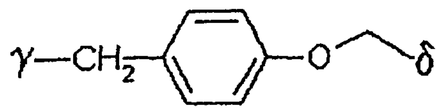

dans lequel γ représente la position de liaison au sucre et δ est la position de liaison au radical X.

6. Procédé selon l'une quelconque des revendications 1 à 5, **caractérisé en ce que** l'on utilise, en tant que catalyseur à transfert de phase, un sel d'ammonium ou de phosphonium quaternaire ou un éther-couronne, de préférence un sel d'ammonium quaternaire.

7. Procédé selon l'une quelconque des revendications 1 à 6, **caractérisé en ce que** l'on utilise, en tant que base, l'hydroxyde de sodium, l'hydroxyde de potassium, le carbonate de césium, le carbonate de potassium, le 1,8-diazabicyclo[5.4.0]undéc-7-ène, le 1,5-diazabicyclo[2.2.2]octane, le t -butoxyde de potassium et le t-butoxyde de sodium.

8. Procédé selon l'une quelconque des revendications 1 à 7, **caractérisé en ce que** la base est ajoutée sous forme solide ou liquide ou en tant que solution à 10 - 70 %.